# EUROPEAN PATENT SPECIFICATION

(11) **EP 3 314 616 B1**
(45) Date of publication and mention of the grant of the patent: **25.08.2021**
(21) Application number: 16813870.9
(22) Date of filing: 24.06.2016
(51) Int. Cl.: G01N 33/531, G01N 33/543, C12N 11/06, C12N 11/14, A61K 9/50, A61K 9/51, B22F 1/00, H01F 1/00

(54) **ENTRAPMENT OF MAGNETIC NANOPARTICLES IN A CROSS-LINKED PROTEIN MATRIX WITHOUT AFFECTING THE FUNCTIONAL PROPERTIES OF THE PROTEIN**
EINSCHLUSS VON MAGNETISCHEN NANOPARTIKELN IN EINER VERNETZTEN PROTEINMATRIX OHNE BEEINTRÄCHTIGUNG DER FUNKTIONALEN EIGENSCHAFTEN DES PROTEINS
CAPTURE DE NANOPARTICULES MAGNÉTIQUES DANS UNE MATRICE DE PROTÉINE RÉTICULÉE SANS INFLUER SUR LES PROPRIÉTÉS FONCTIONNELLES DE LA PROTÉINE

(30) Priority: 26.06.2015 IN 3241CH2015
(43) Date of publication of application: 02.05.2018
(73) Proprietor: Maggenome Technologies PVT. Ltd., Tamil Nadu (IN)
(72) Inventor: IYPE, Tessy, Cochin (Kochi) Kerala 682037 (IN); MOHAN, Sangeetha, Cochin (Kochi) Kerala 682037 (IN); BHATI, Aniruddha, Cochin (Kochi) Kerala 682037 (IN); SANTHOSH, Sreedhar, Cochin (Kochi) Kerala 682037 (IN); CHANIYILPARAMPU, Ramchand Nanappan, Cochin (Kochi) Kerala 682037 (IN)
(74) Representative: Forrest, Stuart
(86) International application number: PCT/IN2016/050199
(87) International publication number: WO 2016/207916

(56) References cited:
- WO-A1-2015/040633
- WO-A1-2015/092106
- WO-A2-2012/023847
- DE-A1- 19 800 294
- JP-A- S59 195 161
- US-A- 5 169 754
- US-A1- 2002 086 441
- US-A1- 2004 146 855
- US-A1- 2005 095 690
- US-A1- 2007 191 594
- US-A1- 2011 229 580
- US-A1- 2013 034 893
- KONERACKA M ET AL: "Direct binding procedure of proteins and enzymes to fine magnetic particles", JOURNAL OF MAGNETISM AND MAGNETIC MATER, ELSEVIER, AMSTERDAM, NL, vol. 252, 1 November 2002 (2002-11-01), pages 409-411, XP004395253, ISSN: 0304-8853, DOI: 10.1016/S0304-8853(02)00595-4
- HUANG SHIH-HUNG ET AL: "Direct binding and characterization of lipase onto magnetic nanoparticles", BIOTECHNOLOGY PROGR, AMERICAN INSTITUTE OF CHEMICAL ENGINEERS, US, vol. 19, no. 3, 1 May 2003 (2003-05-01), pages 1095-1100, XP008096273, ISSN: 8756-7938, DOI: 10.1021/BP025587V
- MADALINA TUDORACHE ET AL: "Strategy of cross-linked enzyme aggregates onto magnetic particles adapted to the green design of biocatalytic synthesis of glycerol carbonate", RSC ADVANCES, vol. 3, no. 12, 1 January 2013 (2013-01-01), page 4052, XP055518083, ISSN: 2046-2069, DOI: 10.1039/c3ra23222k
- BAJPAI A K ET AL: "Investigation of magnetically enhanced swelling behaviour of superparamagnetic starch nanoparticles", BULLETIN OF MATERIALS SCIENCE, INDIAN ACADEMY OF SCIENCES, IN, vol. 36, no. 1, 3 April 2013 (2013-04-03), pages 15-24, XP035372155, ISSN: 0250-4707, DOI: 10.1007/S12034-013-0432-9 [retrieved on 2013-04-03]

## Description

### Technical field of the invention:

The present disclosure relates to entrapped magnetic nanoparticles in a cross linked matrix of an intended protein, without affecting the functional properties of the protein. The disclosure further relates to a method of entrapment of magnetic nanoparticles in a cross linked matrix of an intended protein for use in biological applications.

More particularly, the method disclosed relates to incubating magnetic nanoparticles and the protein of interest together at certain conditions of temperature and pH followed by treatment with a cross linking agent.

### Background of the invention:

Magnetic particles coated with proteins have found immense applications in biotechnology which include (a) isolation and expansion of cells using antibody-coated particles, (b) antibody purification and immunoprecipitation using particles coated with Protein A or G, and (c) immobilization of enzymes among others. Investigators have documented various methods for the preparation of magnetic support systems with diverse physical and biochemical properties [L.R. Witherspoon, S.E.Shuler, S.S.Gilbert, Estimation of Thyroxin, Triiodothyronine, Thyrotropin, Free Thyroxin, and Triiodothyronine Uptake by Use of Magnetic-Particle Solid Phases, Clin. Chem. 31 (1985) 415-419*].* Different types of magnetic materials are available which are based on metals such as Cu, Co, Fe, Ni etc. Magnetic particles are also available as micron and nano sized particles which can be selected based on the specific requirements of the assay. Iron oxide nanoparticles are widely used and have great potential for applications in biology and medicine.

Due to their properties such as super-paramagnetism, low toxicity, high surface area, large surface-to-volume ratio and easy separation under external magnetic fields, iron oxide magnetic nanoparticles have attracted much attention in the past few decades. Different types of biomolecules such as proteins, peptides, enzymes, antibodies, and anticancer agents can be immobilized on these nanoparticles. Magnetic supports for immobilization purpose are either prepared by incorporating magnetic particles during synthesis of the supporting polymer or magnetic particle itself is coated with common support materials such as dextran or agarose.

In most of the commercially available products, proteins are attached to magnetic beads of micron or submicron size which are pre-coated with a stabilizer. For example, Lauva *et. al.,* [Lauva M., Auzans E., Levitsky V and Plavins J, Selective HGMS of colloidal magnetite- binding cells from whole blood. J. of Magnetism and Magnetic Materials, 85 (1990), 295-298] have used heparin-stabilized colloidal magnetite for binding of cells from whole blood. Similarly, dextran-coated magnetite was used as a drug carrier by Rusetski and Ruuge [Rusetski, A.N. and Ruuge, E.K. (1990), Magnetic fluids as a possible drug carrier for thrombosis treatment. J. Magn. Magn.Mater. 85, 299-302]. Witherspoon *et. al.,* (1985) have reported the use of silane-coated ferrite particles for radioimmunoassays.

There are some reports where investigators show that it is possible to bind protein molecules directly on freshly prepared magnetite particles in the presence of cross-linking agents such as Carbodiimide (CDI) [R.V Mehta, R.V Upadhyay, S.W. Charles, C.N. Ramchand, Direct binding of protein to magnetic particles. Biotechnol. Tech. 11 (1997) 493-496]. Koneracka *et. al.,* [Konerackaa M, Kopcanskýa P, Timkoa M, Ramchand CN, de Sequeirac A, Trevand M, Direct binding procedure of proteins and enzymes to fine magnetic particles. J. Mol. Catal. B: Enzym. 18 (2002) 13-18] have used the Carbodiimide method to directly bind several proteins to magnetic particles. Since the magnetic particles are synthesized by the co-precipitation method, it results in the formation of macro-ions. The proposed mechanism of such a binding is that specific adsorption of the amphoteric hydroxyl (-OH) group imparts surface negative charges to the particles in an alkaline medium, and positive charges in an acidic medium. Carbodiimide acts as a linker between the free hydroxyl group on the surface of particles and the -NH₂ groups of the binding protein.

Prior art search revealed a few methods of immobilization of proteins and polysaccharides using different techniques. A patent on 'Formation of Superparamagnetic Particles' (US20040146855) states that their invention features a method for preparing superparamagnetic iron particles by the *in situ* formation of these particles in a cross-linked starch matrix or by the formation of a superparamagnetic chitosan material. The superparamagnetic materials are formed by mild oxidation of ferrous ion, either entrapped into a cross-linked starch matrix or as a chitosan-Fe (II) complex, with the mild oxidizing agent, nitrate, under alkaline conditions.

Another patent (WO2002098364A2) provides novel compositions of binding moiety-nanoparticle conjugates, aggregates of these conjugates, and novel methods of using these conjugates, and aggregates. The nanoparticles in these conjugates can be magnetic metal oxides, either monodisperse or polydisperse. Binding moieties can be, e.g., oligonucleotides, polypeptides, or polysaccharides. Oligonucleotide sequences are linked to either non-polymer surface functionalized metal oxides or with functionalized polymers associated with the metal oxides. The compositions can be used in assays for detecting target molecules, such as nucleic acids and proteins, *in-vitro* or as magnetic resonance (MR) contrast agents to detect target molecules in living organisms.

Another method is described in patent US8420055B2 includes the synthesis of amine functionalized magnetic nanoparticle compositions and processes for synthesizing the same. The process consists of obtaining a carboxylated polymer in substantially pure form, which is used to prepare substantially sized homogeneous, polymer coated carboxyl functionalized magnetic nanoparticle. The carboxyl groups are converted to reactive primary amino groups by the use of a water-soluble carbodiimide followed by reaction of a large excess of a diamine. The amine-terminated nanoparticles are then reacted with bifunctional cross linking agents and with various biomolecules to make nanoparticles for in vitro assays, cell sorting applications and target specific MRI contrast agents.

Patent US6689338B2 described a bio-conjugate including a nanoparticle covalently linked to a biological vector molecule. The nanoparticle is a generally radioactive metal ion and most typically a metal sulfide or metal oxide. The biological vector molecule is typically a monoclonal antibody or fragment of a monoclonal antibody or a peptide having a known affinity to cancer cells. One or more additional, different biological moieties may be covalently linked to the nanoparticle in addition to the biological vector molecule to enhance its activity. The bio-conjugate has utility as an effective radiopharmaceutical to deliver a radiolabel in tumor treatment.

Commonly used methods for binding proteins to magnetic particles require a precoating of the particle with a polymer followed by cross linking of the protein to the coating material. In most current products/ processes the proteins are bound to polymer-coated magnetic particles using a cross linking agent. The extra step of coating of another material on the magnetic particle increases the preparation time of the reagent and the extra layer of polymer might interfere with magnetism.

Hence, there remains a need in the art to provide a less toxic, less time consuming and a convenient way to carry out entrapment of magnetic nanoparticles in the protein matrix, which becomes the objective of the invention, for which protection is sought. Therefore, the inventors of the present invention have come up with a novel method wherein the magnetic particles get entrapped in a cross linked matrix of the protein of interest using an epoxide like Epichlorohydrin as the cross linking agent. This method allows easy separation of the immobilized protein or enzyme from the rest of the reagents. Since this involves direct contact between protein and magnetic particles without any interfering polymeric substances, there will not be any loss in magnetism. Moreover, no change in the functional activity of the enzyme/protein occurs.

Madalina Tudorache et al., "Strategy of cross-linked enzyme aggregates onto magnetic particles adapted to the green design of biocatalytic synthesis of glycerol carbonate", RSC ADVANCES, vol. 3, no. 12, page 4052 discloses a method of preparing biocatalysts based on enzyme immobilization, which leads to the cross-linked enzyme aggregates on magnetic particles.

WO2015040633 discloses a method for extracting biomolecules, preferably proteins, by magnetic particles, wherein the magnetic particles are preferably uncoated magnetic particles, and a process for extracting protein *via* the addition of magnetic particles into a biological system, the application of external magnetic field, collection of a protein-magnetic particle pellet, and resolubilization and finalization of collecting the solublized protein.

US2011229580 discloses compositions, methods and kits for a microsphere with one or more entrapped nanoparticles. The method of preparation comprises atomizing a suspension comprising a polysaccharide and one or more nanoparticles into a solution comprising a cross linking agent.

A K Bajpai et al., "Investigation of magnetically enhanced swelling behaviour of superparamagnetic starch nanoparticles", BULL. MATER. SCI., (2013), Vol. 36, No. 1, pages 15-24 discloses a method for preparing of superparamagnetic nanoparticles of cross-linked starch impregnated homogeneously with nanosized iron oxide, wherein the size of the magnetic polymeric particles is in the range of 20-80 nm.

DE19800294 discloses magnetic particles comprising an inductively heatable magnetic core, completely encapsulated in a polymeric matrix on which ligands are chemically coupled.

### Object of the invention:

The present invention is as described in the accompanying claims.

Herein disclosed is also a method for entrapping magnetic nanoparticles in a cross linked matrix of protein or fragments thereof without affecting the functional properties of the protein.

Herein disclosed are also magnetic nanoparticles entrapped in a cross linked matrix of proteins or fragments thereof for use in biological applications.

### Summary :

Herein disclosed is the entrapment of magnetic nanoparticles in a cross linked matrix of a protein of interest using a cross linking agent. The protein of interest is cross linked to form a matrix in such a way that it facilitates the entrapment of nanoparticles inside, and in tum accomplishes the immobilization of the protein. This is a direct association mechanism wherein the uncoated magnetic nanoparticles without any kind of polymer such as agarose or dextran are used. Also disclosed is a process of entrapment which is achieved when the protein and magnetic nanoparticles are incubated in the presence of a cross linking agent preferably an epoxide and at certain conditions of temperature and pH.

Accordingly, the method for entrapping uncoated magnetic nanoparticles in a cross linked matrix of protein comprises incubating the protein of interest with protein cross-linked with epichlorohydrin, wherein the MNP is entrapped by a method comprising incubating the protein of interest with magnetic nanoparticles in a binding buffer having pH ranging from 6-9 along with a cross linking agent at a temperature of about 4°C to 30°C, with the optional supplementation of salts, and wherein the MNP is entrapped in a ratio ranging from about 1:5 to 1:0.25 and has a particle size in the range of 1 to 100nm,
wherein said kit comprises:
(i) 25µgm to 10mg magnetic nanoparticle entrapped in a matrix of cross-linked protein having affinity to IgG/Fab fragments in 5mM Sodium phosphate buffer, pH 8.0;
(ii) 25ml washing buffer, i.e. IX phosphate buffer pH 8;
(iii) 50ml IgG/Fab fragment elution buffer, i.e. an acidic buffer comprising phosphate buffer pH 2.8; or glycine, pH 2; or L-Arginine, pH 3;
(iv) 2ml neutralizing buffer, i.e. 1M Tris, pH 9.0;
(v) a catalogue comprising instructions and parameters relating to use of the kit;
(vi) proteins selected from the group consisting of ProteinA-MNP, ProteinG- MNP Protein L-MNP, peroxidase-MNP, glutathione-MNP, Bovine serum albumin (BSA)-MNP, ovalbumin-MNP, amylase-MNP, hemoglobin-MNP, lipase-MNP, Fab-MNP, ScFv-MNP, IgG-MNP, lectin-MNP, calmodulin-MNP, streptavidin-MNP, Albumin-MNP, gelatin-MNP, histone-MNP; and
biological samples are selected from the group consisting of biological fluids selected from whole blood, serum, plasma and ascites; cell culture and bacterial cell lysate.

The present application discloses a process of entrapment which is achieved when the protein and magnetic nanoparticles are incubated in the presence of a cross linking agent (i.e. epichlorohydrin) and at certain conditions of temperature and pH.

Accordingly, the method for entrapping uncoated magnetic nanoparticles in a cross linked matrix of protein comprises incubating the protein of interest with magnetic nanoparticles in a binding buffer having pH ranging from 6 to 9 in presence of a cross linking agent for 1-72 hours at a temperature of about 4°C to 30°C, with optional supplementation of salts.

This method is used to prepare magnetic nanoparticle based products which can be used as baits for cell or protein isolation or for generating immobilized enzymes.

Also disclosed is uncoated magnetic nanoparticle (MNP) entrapped in a matrix of protein cross-linked with epichlorohydrin, wherein the said nanoparticle entrapped in protein matrix is in a ratio ranging from about 1:5 to 1:0.25 and has particle size in the range of 1 to 100nm.

Advantageously, the present magnetic nanoparticle-protein conjugate prepared by the presently disclosed method are employed for use in the immobilization, purification and immunoprecipitation of proteins, enzymes, antibodies, antigens, antigenic proteins and fragments thereof, as well as RNA and DNA.

Herein disclosed is a diagnostic kit for the immobilization, identification and purification of IgG, Fab fragments or single chain variants, functional proteins, from a biological sample, the said kit comprising;
(a) 25µgm to 10mg magnetic nanoparticle entrapped in a matrix of cross-linked protein having affinity to IgG/Fab fragments in 5mM Sodium phosphate buffer, pH 8.0;
(b) 25ml washing buffer, i.e. 1X phosphate buffer, pH 8;
(c) 50ml IgG/Fab fragment elution buffer, i.e. an acidic buffer comprising phosphate buffer pH 2.8; or glycine, pH 2 or L-Arginine, pH 3;
(d) 2ml neutralizing buffer, i.e. 1M Tris, pH 9.0;
(e) catalogue comprising instructions and parameters relating to use of the kit.

Accordingly, the diagnostic kit is employed to detect the presence of IgG in biological samples including whole blood, serum, plasma and ascites, cell culture medium and bacterial cell lysate.
Presently disclosed is an immunoprecipitation kit for the detection of an immunogen or antigenic proteins comprising;
(a) 25 µgm to 10mg magnetic nanoparticle entrapped in a matrix of cross-linked protein having affinity to IgG/Fab fragments in 5mM Sodium phosphate buffer, pH 8.0;
(b) primary antibody specific to antigen to be detected; (c) 1ml of 1X Sodium dodecyl (SDS) buffer;
(c) Micro-centrifuge tubes;
(d) Catalogue containing instructions for using kit.

Further, the present disclosure provides magnetic nanoparticles entrapped in a cross linked matrix of proteins for use in biological applications selected from purification of antibodies or antibody fragments, antigenic proteins, functional and structural proteins; enzyme immobilization, antibody immobilization for isolation of different cell types from biological sources; antibody based cell sorting; immunoprecipitation such as protein immunoprecipitation, chromatin immunoprecipitation, RNA immunoprecipitation or similar assays and techniques.

### Brief description of Figures:

**Figure 1****:** Effect of cross linker on immobilization of BSA to magnetic nanoparticles;
**Figure 2****:** Activity of HRP enzyme immobilized on magnetic particles using the cross linker;
**Figure 3**: Activity of free HRP enzyme (unbound/free) measured on three separate days, (Two different dilutions of enzyme (0.5 and 1 U) were used. A and B represents two different experiments conducted on two separate days but under identical conditions);
**Figure 4****:** Immobilization of IgG to magnetic particles by the entrapment method;
**Figure 5****:** Activity of immobilized HRP enzyme (A) and free enzyme (B) measured until 150^{th} day after immobilization;
**Figure 6****:** Improvement in temperature and light sensitivity depicted by activity of immobilized and free enzymes after storage under different experimental conditions. Experimental conditions include storage temperature (Room Temperature and 4°C) and presence or absence of light (uncovered and covered);
**Figure 7****:** Purification of whole IgGs from human blood plasma using Protein A-MNPs (Magnetic Nanoparticles) by Non-Reducing SDS-PAGE analysis; **[Expt.1:** Purification of IgG from 50 µl of blood plasma. **Expt.2:** Same as Expt.1. **Expt.3:** Purification of IgG reusing Protein A-MNPs from Expt.1.] E1-E3: Eluted IgG;
**Figure 8****:** Purification of IgG from cell culture supernatant using Protein A-MNPs; E1-E4: IgG eluted in L-Arginine in four steps;
**Figure 9****:** Purification of a Fab from bacterial lysate using Protein A-MNPs; E1-E2 represent Fabs eluted in two steps and M indicates the standard protein marker
**Figure 10****:** Antibody binding capacity of Protein A-MNPs in an immunoprecipitation (IP) experiment;
**Figure 11****:** Co-Immunoprecipitation of SREBP cleavage-activating protein (SCAP) and Sterol regulatory element-binding transcription factor 1a (SREBP-1a) Proteins using Protein A-MNPs, wherein M is the marker, A is the Negative control, B: IP with protein A-MNP and C is the IP with protein A-Sepharose;
**Figure 12****:** Determining the GST binding capacity of Glutathione-MNPs: Binding and elution of different amounts of GST enzyme, 1-7 at the top of the figure (from left to right) indicate the different amounts of eluted GST;
**Figure 13****:** Purification of a GST-tagged recombinant protein from bacterial culture using glutathione-MNPs;
**Figure 14****:** TEM image of the magnetic nanoparticles synthesized by the present method: (A) Uncoated Magnetic Nanoparticles and (B) Uncoated magnetic Nanoparticles entrapped in a matrix of cross-linked protein;
**Figure 15****:** Easy separation of immobilized proteins using a magnetic stand.

### Detailed description:

The invention will now be described in detail in connection with certain preferred and optional embodiments, so that various aspects thereof may be more fully understood and appreciated.

As used herein, the terms the methods of entrapment including 'immobilization' and 'crosslinking' have been interchangeably employed in the present invention. The present disclosure describes the entrapment of uncoated magnetic nanoparticles i.e. magnetic nanoparticles in the absence of a polymer in a cross linked matrix of a protein of interest using a cross linking agent.

The technique described in the present disclosure is novel as it demonstrates for the first time, a method to immobilize a protein by cross linking itself and meanwhile trapping the nano-sized magnetic particles within the protein matrix. Cross linking is accomplished in the presence of a cross linking agent.

The disclosure provides a method for the entrapping of uncoated magnetic nanoparticles in a cross linked matrix of protein, the said method comprising incubating the protein of interest with magnetic nanoparticles in a binding buffer having pH ranging from 6-9 along with a cross linking agent for 1-72 hours at a temperature of about 4°C to 30°C, with the optional supplementation of salts.

Keeping with the above disclosure, the magnetic nanoparticles are selected from the group consisting of oxides of synthetic analogues of any suitable magnetic material or combination of materials selected from the group consisting of magnetite, ulvospinel, hematite, ilmenite, maghemite, jacobsite, trevorite, magnesioferrite, pyrrhotite, greigite, troilite, goethite, lepidocrocite, feroxyhyte, iron, nickel, cobalt, awaruite, wairauite, or any combination thereof.

The suitable cross linking agent is Epichlorohydrin.

The present disclosure provides the entrapment of proteins selected from functional proteins such as enzymes, antibody molecules, antigenic proteins, peptide fragments and other structural proteins, including combinations and variations thereof.

Enzymes are selected from peroxidases, amylases, pectinases, esterases, proteases, lipases, ligases, transferases, synthases, hydrolases, oxido-reductases and isomerases. Anti-oxidants selected from glutathione, catalases, superoxide dismutases and the like. Antibodies include full length IgGs, Fab fragments of antibodies, single-chain variable fragments (scFvs) and variations thereof Immunogens or antigenic proteins, immuno- globulin binding proteins such as bacterial proteins including protein A, protein G, and protein Land other allergens and other proteins including but not limited to histones, fetuins, pepstatin etc. Carrier proteins or transport proteins include membrane transport proteins, bovine serum albumin (BSA), myoglobin, cytochromes, ovalbumin, hemoglobin and other relevant proteins. Functional proteins are selected from but not limited to bacterial proteins such gelatin, histones and combinations thereof and any variation of structural proteins or combinations thereof. Structural proteins may be selected from gelatin, collagen, fibronectin and laminin, keratins, actin, actinin, cadherins, clathrins, elastin, vitronectin, vimetin and the like and combinations thereof and any variation of structural proteins or combinations thereof. The aforesaid proteins entrapped by the present method are either prepared by recombinant methods or are native proteins or fragments thereof.

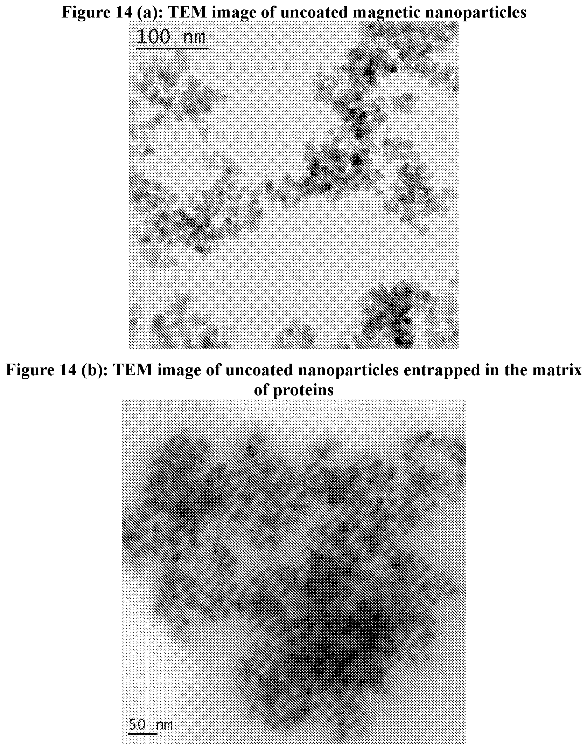

The uncoated magnetic nanoparticles employed in the present disclosure have a mean particle size ranging from 1 to 100 nm. Transmission electron microscopy (TEM) analysis of nanoparticles and nanoparticles entrapped within the cross linked protein matrix are shown in Figure 14 (a) and (b).

In a subsequent disclosure, the magnetic nanoparticles cross-linked in the matrix of protein have a mean particle size in the range of 1nm to 30nm. Accordingly, TEM imaging in Figure 14(b) shows particle size of uncoated magnetic nanoparticles entrapped in a matrix of cross-linked protein to be preferably in the range of 1 to 20nm. They also possess greater surface area per weight as compared to micron sized particles.

Discloses is a method wherein to a specified amount of magnetic nanoparticles, the required amount of protein was added and cross linking was carried out in the presence of Epichlorohydrin. The reaction was carried out in 5-50mM Phosphate buffer at a pH range of 6.0-9.0. The mix was then incubated for 18 to 24 hrs at a temperature of 4°C to 30°C with continuous shaking. An additional 24 to 48 hrs of incubation at 4°C to 30°C without rotation was also included to facilitate stability to the bonds formed between cross linker and the protein.

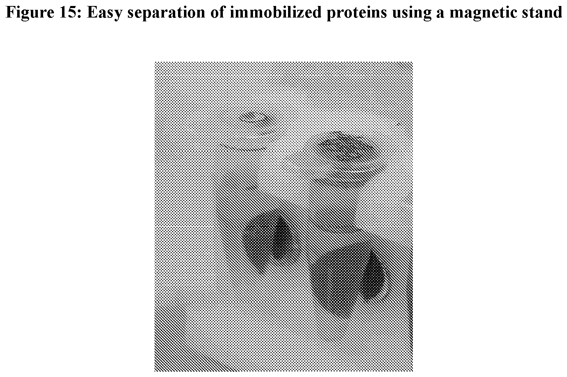

After immobilization, the beads were separated and washed using a magnet. Figure 15 shows separation of the immobilized protein obtained by the presently disclosed method. Both immobilized and free protein concentrations were measured using a standard protein assay method and the percentage of immobilization was calculated. In the case of enzymes, activity of the enzyme was measured by performing standard assays. In the case of Glutathione, estimation was done using standard glutathione assay. Other preferable protein assay methods that may be used include Bradford's assay method and Modified Lowry's method.

In one more disclosure, the concentration of epichlorohydrin employed in the present invention is having a concentration in the range of about 0.1M to about 2M. More preferably, a concentration of about 0.6 M to about 1.2M is employed in the present disclosure. Further, the binding buffer is selected from a Phosphate, Carbonate, Borate and combinations thereof, with molar concentrations ranging from 5 mM to 200 mM. Salts which may be optionally added are selected from sodium chloride, potassium chloride, calcium chloride, magnesium chloride and any combination thereof.

The present disclosure provides magnetic nanoparticle (MNP) cross-linked in the matrix of protein in a ratio ranging from about 1:5 to 1:0.25. Accordingly, MNP to protein ratio in the crosslinked matrix is 1:5, 1:4, 1:1, 1:0.5 and 1:0.25.

The present disclosure is to uncoated magnetic nanoparticle (MNP) entrapped in a matrix of protein cross-linked with epichlorohydrin, wherein said nanoparticle entrapped in protein matrix is in a ratio ranging from about 1:5 to 1:0.25 and has particle size in the range of 1 to 100nm.

Accordingly, the magnetic nanoparticles cross linked with preferable proteins are selected from the group consisting of but are not limited to Protein A- MNP, Protein G-MNP, protein L-MNP, peroxidase-MNP, glutathione-MNP, Bovine serum albumin (BSA)-MNP, ovalbumin-MNP, amylase-MNP, hemoglobin-MNP, lipase-MNP, Fab-MNP, ScFv-MNP, IgG-MNP, lectin-MNP, calmodulin-MNP, streptavidin-MNP, Albumin-MNP, gelatin-MNP, histone-MNP and others.

The uncoated magnetic nanoparticles employed in the magnetic nanoparticles cross linked with preferable proteins are selected from the group consisting of magnetite, ulvospinel, hematite, ilmenite, maghemite, jacobsite, trevorite, magnesioferrite, pyrrhotite, greigite, troilite, goethite, lepidocrocite, feroxyhyte, iron, nickel, cobalt, awaruite, wairauite, or any combination thereof.

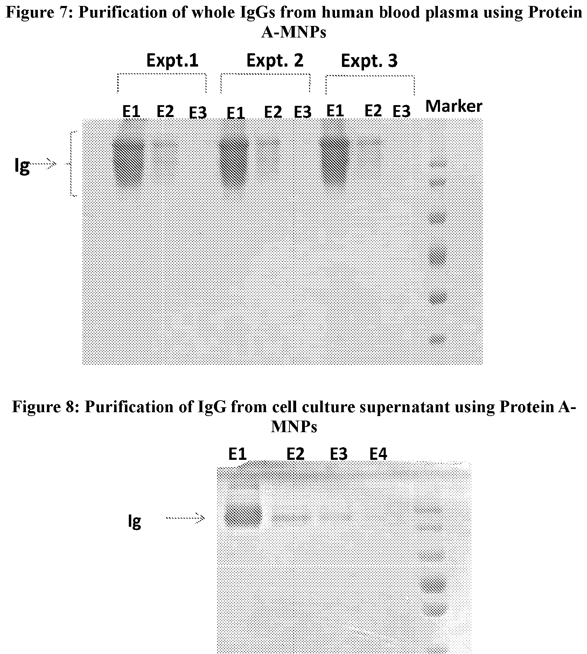

Herein disclosed is IgG binding immunogenic proteins crosslinked with magnetic nanoparticles by the present method. The antibody molecules bind Protein A during incubation and get eluted with an acidic elution buffer. The magnetic particles act as a support system and facilitate easy separation of the purified antibody by placing the reaction tube on a magnetic stand. The presence of the purified antibody was visualized by SDS-PAGE analysis under non-reducing conditions as observed in Figure 7.

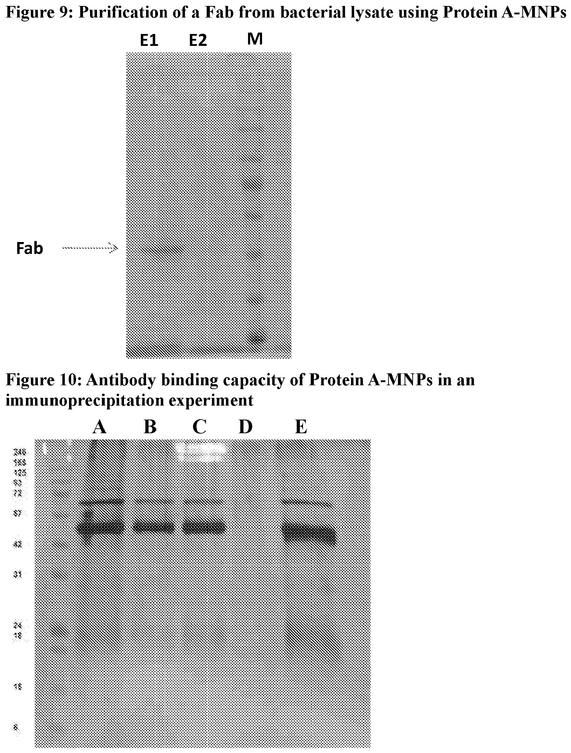

Protein A-MNP was used for purification of IgG molecules from culture media of mammalian cells transiently transfected with an expression vector which lead to production and extracellular secretion of IgG molecules. Presence of purified antibody was visualized by SDS-PAGE analysis under non-reducing conditions **(****Figure 8****)**. Protein A-MNP synthesized by the presently disclosed method was used for purification of Fab *(fragment-antigen binding)* fragment of antibody. Bacterial cells expressing recombinant Fab molecules were lysed and incubated with Protein A-MNPs to obtain Fab molecules bound to Protein A-MNPs **(****Figure 9****)**.

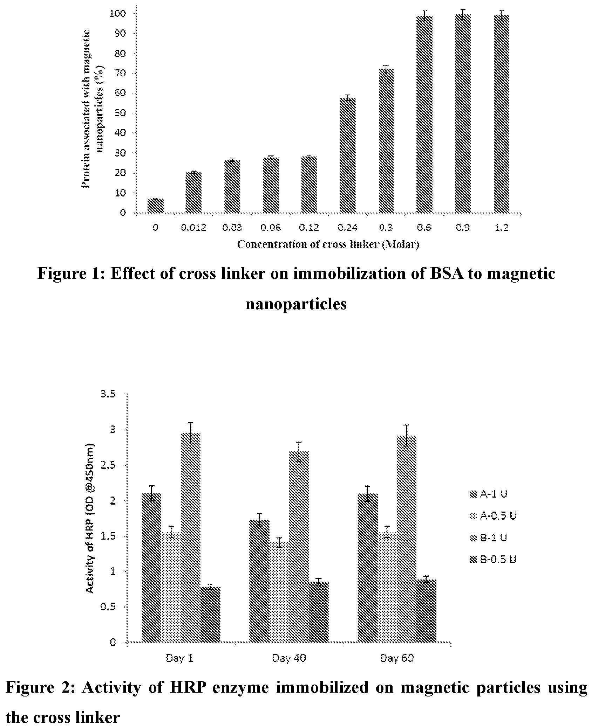

Also disclosed is the immobilization of Bovine Serum Albumin (BSA) on magnetic nanoparticles, wherein immobilization of BSA to magnetic particles was very weak in the absence of epoxide and also at low concentrations of epoxide. Percentage of association increased when 0.24M epoxide was used and progressively increased with increasing amounts. A saturation level was attained using the said cross-linking agent in the range of 0.6M - 1.2M **(****Figure 1****)**.

Further disclosed is immobilization of Horse Radish Peroxidase (HRP) on to magnetic nanoparticles wherein the enzyme activity was measured after immobilization on three separate time points (Day 1, 40 and 60). Two different dilutions of enzyme (0.5 and 1 U) were used. A and B represent two different experiments conducted on two separate days but under identical conditions. Y-axis represents the intensity of Yellow color developed from TMB substrate measured at 450nm. The X-axis represents the activity of the enzyme given in units. It is clear that the activity of the immobilized enzyme remains intact even after 60 days **(****Figure 2****).**

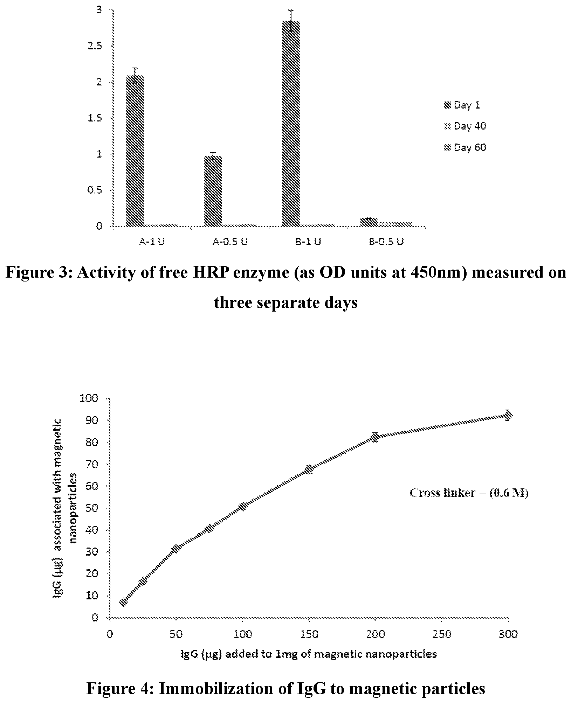

Disclosed is the result of an enzyme activity assay (TMB assay) for HRP enzyme which is present in solution (not immobilized). The enzyme activity was measured on three separate time points (Day I, 40 and 60). This experiment was done in parallel with the assay of immobilized enzyme. Two different dilutions of enzyme (0.5 and 1 U) were used. A and B represents two different experiments conducted on two separate days but under identical conditions. Y-axis represents intensity of Yellow color developed from TMB substrate measured at 450 nm. X-axis represents activity of the enzyme given in units. Unlike the immobilized enzyme, the activity of the free enzyme was lost after storage at 4°C for several days **(****Figure 3****).**

In yet another disclosure is the immobilization of a full length IgG to magnetic nanoparticles at the aforementioned immobilization conditions. Accordingly, increasing amounts of IgG along with BSA was added to
1mg of nanoparticles in the presence of 0.6M epoxide. Since the IgG is conjugated to HRP, the amount of IgG immobilized was determined based on the enzyme activity. It is clear from **Figure 4** of progressive increase in immobilization but was not yet saturated even when 300µg antibody was added.

Presently disclosed is a diagnostic kit for the immobilization, purification and identification of IgG, Fab fragments or single chain variants, functional proteins, from biological fluids, the said kit comprising;
(a) 25µgm to 10mg magnetic nanoparticle entrapped in a matrix of cross-linked protein having affinity to IgG/Fab fragments in 5mM Sodium phosphate buffer, pH 8.0;
(b) 25ml washing buffer, i.e. 1X phosphate buffer pH 8;
(c) 50ml IgG/Fab fragment elution buffer, i.e. an acidic buffer comprising phosphate buffer pH 2.8; or glycine, pH 2; or L-Arginine, pH 3;
(d) 2ml of a neutralizing buffer, i.e. 1M Tris, pH 9.0;
(e) catalogue comprising instructions and parameters relating to use of the kit.

The proteins that are cross linked in the presence of the magnetic nanoparticles include IgG binding proteins such as Protein A, Protein L, Protein G, other functional proteins such as enzymes and anti-oxidants such as glutathione, peroxidases; immunoglobulins such as IgG, IgE, IgA; Fab fragments; scFv fragments; structural proteins, including combinations and variations thereof.

The Protein A-MNPs are previously prepared by cross linking approximately 10 mg of MNPs in 1ml of 50 mM Phosphate buffer, pH 8 with 4-5 mg of Protein A prepared in the same buffer in the presence of the chemical cross linking agent, i.e. epichlorohydrin. The cross linking was performed at 4°C for 48-72hrs with optional rotation and optional supplementation of salts.

The diagnostic kit is employed to detect presence of IgG in biological samples including whole blood, serum, plasma and ascites, cell culture medium and bacterial cell lysate.

Disclosed herein is the method of using the diagnostic kit for identification and purification of IgG, Fab fragments or single chain variants, the said method comprising; (i) diluting blood plasma with phosphate buffer and treating it with the magnetic nanoparticle entrapped in a matrix of cross-linked protein synthesized by the present process and subsequently subjecting the sample to rotation for about 2hrs at room temperature;
(ii) washing magnetic particles after rotation in step (i) at least twice with phosphate buffer and eluting the antibody bound to the Protein A-magnetic nanoparticles with an acidic buffer;
(iii) neutralizing the antibody with 1M Tris buffer, pH9 and characterizing the IgG by gel electrophoresis technique followed by Western blotting.

The diagnostic kit is more preferably stored at 4°C and protected from light. However, it can be stored at higher temperatures up to 30°C and in presence of light without affecting the magnetic properties of the protein crosslinked in nanoparticles. In accordance with the above disclosure, Protein A-MNP prepared by the present method was used for purification of IgG molecules from blood plasma. Precisely, 50 µl of blood plasma was diluted with 850 µl of phosphate buffer and mixed with 100 µ1 of Protein A-MNPs. It was then mixed thoroughly by gentle rotation for 2 hrs at room temp. The supernatant was discarded and the pellet was washed with phosphate buffer repeatedly. The bound antibody was eluted with an acidic buffer (Phosphate buffer, pH 2.8; or Glycine, pH 2; or L-Arginine, pH 3 or any other buffer that has been described for IgG elution). Pure antibody was then neutralized using an appropriate buffer (1M Tris, pH 9.0). The presence of IgG in the eluate was visualized by performing SDS- PAGE under non-reducing conditions followed by Coomassie staining of the gel. Figure 7 depicting SDS-PAGE shows the Protein A-MNP prepared by the present method is capable of purifying IgG molecules from blood plasma and that the said Protein A-MNPs can be reused for subsequent purifications.

Employing an almost similar method of using the diagnostic kit for identification and purification of IgG and Fab fragments, the present inventors disclose the purification and identification of the said fragments from mammalian cell culture systems as well as bacterial lysates. Figure 8 depicts the identification and purification on an SDS gel, wherein the monoclonal antibody produced using transfected mammalian cell lines was purified using the kit comprising the Protein A-MNPs synthesized by the present process.

Fab molecules bound to Protein A-MNPs were obtained by the present method. Figure 9 depicts the presence of Fab molecule having a molecular weight of ∼48kDa on SDS-PAGE under non-reducing conditions.

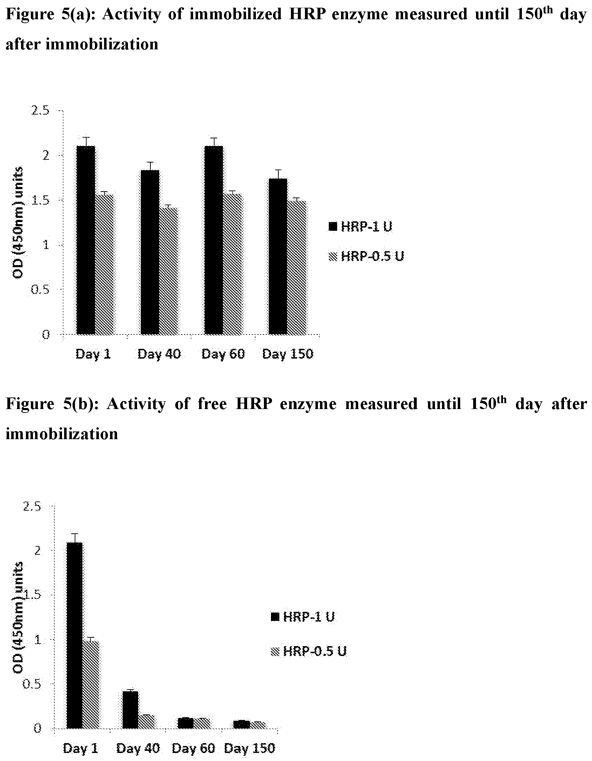

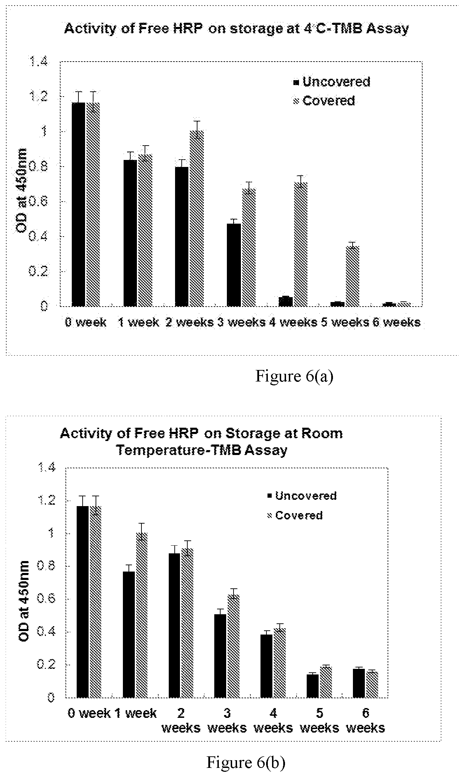

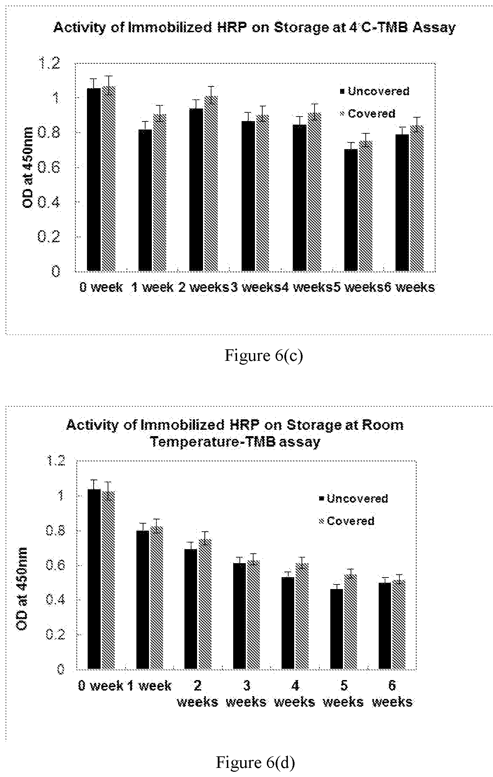

As an extension of data provided as Figure 2 and Figure 3, the enzyme activity of free and immobilized HRP were measured on different days until the 150^{th} day after immobilization **(****Figure 5** **A, B).** Two different dilutions of enzyme (0.5 and 1 U) were used. Y-axis represents the intensity of yellow color developed from TMB substrate measured at 450nm. The X-axis represents the days of measurement. It is clear from Figure 5(a) that the activity of the immobilized enzyme remains intact even after 150 days while no activity of free enzyme remains. Both immobilized and free enzymes were stored at 4°C. Further, Figure 5(b) shows enhanced activity of the HRP enzyme immobilized by the present method compared to the free enzyme. Additionally, enzymatic activity of immobilized and free enzymes was compared under different storage conditions such as room temperature and 4°C for testing temperature sensitivity as well as in the presence and absence of light represented as uncovered and covered. The activity of enzyme was measured once a week from 1^{st} to 6^{th} week from the day of immobilization. **(****Figure 6 A-D****).** Y-axis represents intensity of yellow color developed from TMB substrate measured at 450nm. X-axis represents the weeks of measurement. It is evident from the results that the activity of immobilized enzyme is preserved much efficiently compared to the free enzyme under any given experimental conditions.

In yet another disclosure is the selective binding ability of the present protein-magnetic nanoparticles to an antigenic protein.

An immunoprecipitation (IP) experiment was performed using Protein A-MNP synthesized by the presently disclosed method. A primary antibody was used to selectively bind the antigen of interest present in the cell lysate. This antibody along with the target antigen is captured by Protein A. Immunoprecipitation indicated that Protein A-MNP has the same antibody binding capacity as Protein A-Sepharose, a well-documented medium for immunoglobulin purification and fractionation, and is suitable for immunoprecipitation. Figure 10 indicates that expression of the antibody-Protein A-MNP conjugate formed, wherein binding ability of antibody to protein A is observed to be similar to that of protein A-Sepharose.

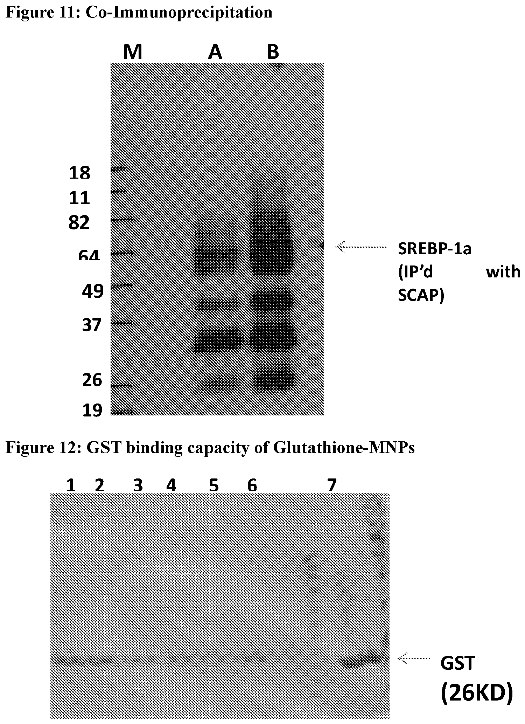

Additionally an IP experiment was performed using the present novel Protein A-MNPs. This experiment was a co-immunoprecipitation in which the association between two proteins was studied. RIPA buffer lysates of ovary were used. Primary antibody against SCAP protein was used in the IP phase and an antibody against Sterol Regulating element binding protein 1-a (SREBP-Ia) was used in western blot. A primary antibody against a SCAP protein (SREBP - cleavage activating protein) was used to selectively bind the protein complex from the RIPA lysate prepared using ovarian tissue. Protein A-MNP was used to bind to the antibody along with the protein complex. The antibody-protein complex was then eluted from the Protein A-MNPs by boiling in 1X SDS sample buffer and separated by running the samples on SDS-PAGE. The proteins present on the gel were then transferred to a nitrocellulose /PVDF membrane by Western blotting. Presence of the interacting protein was confirmed using another primary antibody against SREBP-la. The Protein A-MNPs gave the same performance as Protein A-Sepharose (**Figure 11**).

Accordingly, the present disclosure is also to an immunoprecipitation kit comprising;
(a) 25µgm to 10mg magnetic nanoparticle entrapped in a matrix of cross-linked protein having affinity to IgG/Fab fragments in 5mM Sodium phosphate buffer, pH 8.0;
(b) primary antibody specific to antigen to be detected; (c) 1ml of 1X Sodium Dodecyl Sulfate (SDS) buffer; (d) Micro-centrifuge tubes; and
(c) Catalogue containing instructions for using kit.

Further, the method of employing the immunoprecipitation kit comprises; (i) treating a cell lysate with primary antibody specific to the protein depending on the requirements of the experiment to be detected followed by addition of magnetic nanoparticle entrapped in protein to form an Ag-primary antibody-protein-MNP complex; and (ii) heating the Ag-primary antibody-protein-MNP complex of step (i) up to 100°C, followed by running the mixture on SDS PAGE under reducing conditions to identify the Ag.

In the aforesaid diagnostic kit and immunoprecipitation kit the magnetic nanoparticle entrapped in a matrix of cross-linked protein are selected from a wide range of proteins such as functional proteins selected from enzymes, antibody molecules, antigenic proteins, peptide fragments and other structural proteins, including combinations and variations thereof. The magnetic nanoparticles entrapped are selected from magnetite, ulvospinel, hematite, ilmenite, maghemite, jacobsite, trevorite, magnesioferrite, pyrrhotite, greigite, troilite, goethite, lepidocrocite, feroxyhyte, iron, nickel, cobalt, awaruite, wairauite, or any combination thereof.

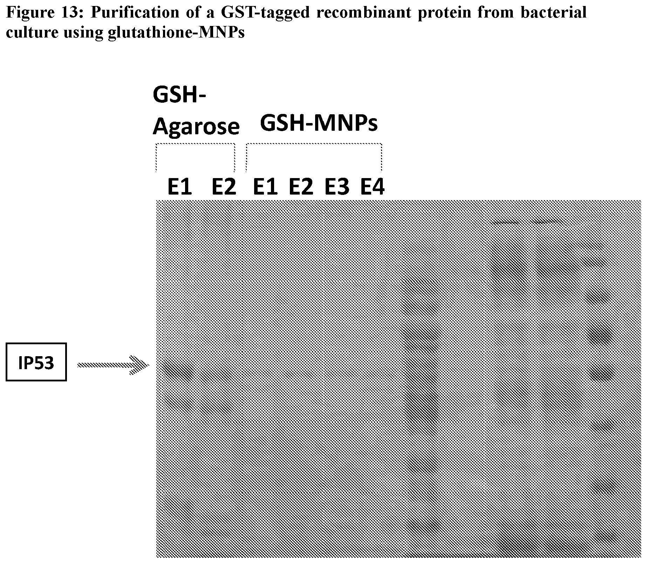

In another disclosure, magnetic nanoparticles are entrapped by the present method in glutathione (GSH), for the detection and purification of GST tagged fusion proteins. The magnetic particles and the peptides were incubated in presence of the cross-linker. The Glutathione-MNPs were tested by purifying GST (Glutathione S Transferase) enzyme since Glutathione is a substrate of GST. GST in solution can bind to immobilized GSH which can later be eluted with buffer containing excess GSH. The presence of GST enzyme in the eluate was visualized by SDS-PAGE analysis **(****Figure 12****).** Similarly, Glutathione-MNPs were used for purification of GST-tagged fusion protein **(****Figure 13****).**

The present disclosure provides magnetic nanoparticles entrapped in a cross linked matrix of proteins for use in biological applications selected from purification of antibodies or antibody fragments, antigenic proteins, functional and structural proteins; enzyme immobilization, antibody immobilization for isolation of different cell types from biological sources; antibody based cell sorting; immunoprecipitation experiments such as protein immunoprecipitation, chromatin immunoprecipitation, RNA immunoprecipitation or similar assays and techniques. Magnetic nanoparticle entrapped in proteins by the present process can be employed in several biological applications, wherein the conjugation of magnetic nanoparticles with the protein is required.

Advantageously, the present method is less time consuming and economical. This is a direct entrapment mechanism which does not include any kind of polymeric material as a coating on the surface of the magnetic particles. Instead the protein is in direct association with particles and hence the magnetic property of the particle is not diminished. Using this method the functional activity of the protein is not lost even after immobilization.

Some typical examples illustrating the present disclosure are provided; however, these are exemplary only and should not be regarded as limiting the elements of the present disclosure.

### Examples

### Example: Bovine Serum Albumin (BSA) immobilization on magnetic nano particles

To 10 mg of Fe₃O₄ iron oxide magnetic nanoparticles in 5mM Sodium phosphate buffer (pH 8.0), 2.5mg of BSA was added and cross linking was carried out in presence of epichlorohydrin having a concentration of 1M. The mix was then incubated for 20 hrs at room temperature with continuous shaking. After incubation, beads were separated and washed using a magnet. Both immobilized and free protein concentrations were measured using Bradford's reagent and the percentage of association was calculated (**Figure 1**).

### Example 2: Horse Radish Peroxidase (HRP) enzyme immobilization on magnetic nanoparticles

To 1 mg of hematiteFe₂O₃magnetic nanoparticles in 5mM Sodium phosphate buffer (pH 8.0), approximately 1U and 0.5 U of HRP enzyme (mixed together with 200µg of BSA) was added and cross linking was carried out in the presence of 0.6 M Epichlorohydrin. The mix was then incubated for 20 hrs at 4°C with continuous shaking. After incubation, the beads were separated and washed using a magnet. Both immobilized and free protein concentrations were measured and the percentage of association was calculated. Protein estimation assay by Bradford's method showed almost 90% association of enzyme with nanoparticles (immobilization). Enzyme activity in both immobilized and free fractions were also measured. When using small amounts of enzymes to bind, BSA was used as a diluent and stabilizer. The activity of the enzyme which is immobilized on magnetic particles is retained even after long periods of storage at 4°C as shown by subsequent assays **(****Figure 2** **and** **3****)**.

### Example 3: Immobilization of antibodies (full length IgG) on to magnetic nanoparticles

To 1 mg of Nickel (Ni) magnetic nanoparticles in 5mM Sodium phosphate buffer pH 8.0, 10-300 µg of antibody along with 250 µg of BSA was added and cross linking was carried out in the presence of Epichlorohydrin (0.6M). The mix was then incubated for 20 hours at 4°C with continuous shaking. After incubation, the beads were separated and washed using a magnet. Both immobilized and free protein concentrations were measured and the percentage of association was calculated. When using small amounts of antibodies, BSA can be used as a diluent and stabilizer (**Figure 4**).

### Example 4: Immobilization of HRP enzyme and demonstration of retention of activity under different experimental conditions

To 1 mg of Cobalt (Co) magnetic nanoparticles in 5mM Sodium phosphate buffer pH 8.0, approximately 1U and 0.5 U of HRP enzyme (mixed together with 200µg of BSA) was added and cross linking was carried out in the presence of 0.6M Epichlorohydrin. The mix was then incubated for 20 hours at 4°C with continuous shaking. After incubation, the beads were separated and washed using a magnet. Both immobilized and free protein concentrations were measured at different time points until the 150^{th} day after immobilization. The activity of enzyme was measured by performing a TMB assay and the yellow color developed was measured at 450nm (**Figure 5 A-B**). In order to compare light sensitivity and temperature sensitivity of immobilized and free enzyme the following experiment was done. Aliquots of immobilized and free enzymes were stored at room temperature and at 4°C either covered in foil or uncovered. Equal concentrations of immobilized and free enzymes were stored under all these conditions were used for the TMB assay. The yellow color developed was measured at 450nm **(****Figure 6 A-D****).** The X-axis represents the weeks of measurement and the Y-axis represents OD units at 450nm. Activity of free enzyme stored at 4°C (A) and at room temperature (B) was either completely lost or diminished significantly after 6 weeks whether tubes were kept covered or uncovered. But the immobilized enzyme maintained 50-60% activity at room temperature (C) and more than 80% activity at 4°C (D) either covered or uncovered.

### Example 5: Immobilization of Protein A on Magnetic nanoparticles and use of Protein A-MNP for purification of antibodies (IgGs) from blood plasma

Protein A-MNPs (Fe₃O₄ iron oxide) were prepared by cross linking approximately 10 mg of MNPs in 1ml Phosphate buffer (50 mM, pH 8) with 4-5 mg of Protein A (prepared in the same buffer) in the presence of the chemical cross linking agent preferably an epoxide such as Epichlorohydrin (1M). The cross linking was performed at 4°C for 24-72 hrs with and without rotation in the presence or absence of NaCl. Non-specific binding to the MNP's can be reduced by blocking in either 1M Tris, pH 9.5 or 1M Glycine, pH 9.5 for 24 hrs at 4°C if blocking becomes necessary. Protein A-MNP thus prepared was used for the purification of IgG molecules from blood plasma. Precisely, 50 µl of blood plasma was diluted with 850 µl of phosphate buffer pH 8 and mixed with 100 µl of Protein A-MNPs.

It was then mixed thoroughly by gentle rotation for 2 hrs at room temp. The supernatant was discarded and the pellet was washed with phosphate buffer repeatedly. The bound antibody was eluted with an acidic buffer (Phosphate buffer, pH 2.8; or Glycine, or pH 2; L-Arginine, pH 3 or any other buffer that has been described for IgG elution). The pure antibody was then neutralized using an appropriate buffer (1M Tris, pH 9.0). The presence of IgG in the eluate was visualized by performing SDS-PAGE under non-reducing conditions followed by Coomassie staining of the gel **(****Figure 7****).**

### Example 6: Immobilization of Protein A on Magnetic nanoparticles and the use of Protein A-MNP for the purification of IgG from cell culture medium (supernatant)

Protein A-MNPs were prepared as described in Example 5. 1ml of Protein A-MNPs was added and mixed overnight with 125ml of cell culture medium into which the IgGs were secreted by mammalian cells transiently transfected with an expression vector which expresses IgG After incubation, the spent medium was removed and the MNPs were washed with IX PBS multiple times and finally eluted the IgGs with an acidic buffer preferably L-Arginine, pH 3.0. The pure antibody was then neutralized using an appropriate buffer preferably 1M Tris buffer pH 9.0. The presence of IgG in the eluate was visualized by performing SDS-PAGE under non-reducing conditions followed by Coomassie staining of the gel **(****Figure 8****).**

### Example 7: Immobilization of Protein A on Magnetic nanoparticles and the use of Protein A-MNP for the purification of Fab fragment of IgG from bacterial culture medium

Protein A-MNPs were prepared as described for Example 5. 1ml of Protein A-MNPs was added and mixed overnight with 100 ml of bacterial cell lysate which contains the recombinant Fab molecule. After incubation, the spent lysate was removed and the MNPs were washed with IX PBS multiple times and finally eluted the Fab with an acidic buffer preferably but not limited to Phosphate buffer, pH 2.8. The pure Fab was then neutralized using an appropriate buffer. The presence of Fab in the eluate was visualized by performing SDS-PAGE under non-reducing conditions followed by Coomassie staining of the gel **(****Figure 9****).**

### Example 8: Immobilization of Protein A on Magnetic nanoparticles and the use of Protein A-MNP for immunoprecipitation

In immunoprecipitation (IP), a primary antibody is used to selectively bind the antigen of interest present in the cell lysate. This primary antibody will be captured by Protein A along with the target antigen. An experiment (Figure 10) was performed to prove that the Protein A-MNP has the same antibody binding capacity as the leading competitor product, Protein A-Sepharose. The antibody bound to MNPs was eluted by boiling in IX SDS sample buffer and the samples were resolved on SDS-PAGE under reducing conditions followed by silver staining. Additionally, an IP experiment was performed using the present novel Protein A-Fe₂TiO₄ produced by the present method. This experiment was a co-immunoprecipitation in which the association between two proteins was studied. A primary antibody against a SCAP protein (SREBP cleavage activating protein) was used to selectively bind the protein complex from the RIPA lysate prepared using ovarian tissue. 50 µl of Protein A-MNP was used to capture the antibody along with the protein complex. The antibody-protein complex was then eluted from the Protein A-MNPs by boiling in IX SDS sample buffer and separated by running the samples on SDS-PAGE under reducing conditions. The proteins present on the gel were then transferred to a nitrocellulose /PVDF membrane by Western blotting. The presence of the interacting protein was confirmed using another primary antibody against SREBP-1a and it proves that there is an association between the two proteins. The Protein A-MNP gave the same performance as Protein A-Sepharose **(****Figure 11****).**

### Example 9: Immobilization of Glutathione on Magnetic nanoparticles and the use of Glutathione-MNPs for purification of GST-tagged fusion proteins

The immobilization of the peptide-Glutathione (GSH) was performed using the entrapment method as previously described. The magnetic particles i.e. Fe₃O₄ and the peptides were incubated in the presence of a cross linker which is preferably an epoxide such as Epichlorohydrin. The performance of Glutathione-MNPs was tested by purifying GST (Glutathione S Transferase) enzyme. Since Glutathione is its substrate, the GST enzyme can bind to the immobilized GSH which can later be eluted with a buffer containing excess amount of GSH. The presence of GST enzyme in the eluate was visualized by SDS-PAGE analysis **(****Figure 12****).** Similarly the Glutathione-MNPs were used for the purification of a GST-tagged fusion protein by incubating approximately 500µl of MNPs in 50ml bacterial cell lysate which contains an over-expressed recombinant GST-tagged protein. The elution of purified protein was carried out using a buffer containing excess glutathione **(****Figure 13****).**

### Example 10: Diagnostic kit for immobilization, purification and identification

The present inventors designed a diagnostic kit for the immobilization, purification and identification of IgG, Fab fragments or single chain variants from biological fluids. The kit comprised (a) 25 µgm to 10mgof Fe₃O₄ cross-linked with Protein A having affinity to IgG/Fab fragments in 5mM Sodium phosphate buffer, pH 8.0; (b) 25ml of washing buffer, i.e. IX phosphate buffer) pH-8;(c) 50ml of IgG/Fab fragment elution buffer, i.e. an acidic buffer comprising phosphate buffer pH 2.8; or glycine, pH 2;or L-Arginine, pH 3; (d) a neutralizing buffer, i.e. 1M Tris, pH 9.0; and (e) a catalogue comprising instructions and parameters relating to use of the kit.50 µl of blood plasma was diluted with 850 µl of phosphate buffer and mixed with 100 µl of Protein A-MNPs. It was then mixed thoroughly by gentle rotation for 2 hrs at room temp. The supernatant was discarded and the pellet was washed with phosphate buffer repeatedly. The bound antibody was eluted with an acidic buffer (Phosphate buffer, pH 2.8; Glycine, pH 2; L-Arginine, pH 3 or any other buffer that has been described for IgG elution). The pure antibody was then neutralized using an appropriate buffer (1M Tris, pH 9.0). The presence of IgG in the eluate was visualized by performing SDS-PAGE under non-reducing conditions followed by Coomassie staining of the gel.

### Industrial advantages of the present invention:

1. Absence of polymeric coating of the magnetic nanoparticle renders the protein in direct contact with the nanoparticle therefore the magnetic property of the protein is not reduced.
2. The presently synthesized magnetic nanoparticles entrapped in protein can be used in a large range of diagnostic procedures, by entrapping the said nanoparticle in the protein of interest.
3. Procedures such as Immobilization of enzymes, immunoprecipitation and identification and purification of immunogenic proteins are conveniently and easily performed using the present method. Immobilized enzymes can be used to overcome inborn metabolic disorders by the supply of immobilized enzymes. The entrapment technique can be effectively used in drug delivery systems especially to oncogenic sites.
4. The enzyme immobilized onto magnetic nanoparticle are found to withstand harsh experimental conditions including variation in temperature, exposure to light and presence of organic solvents among others.
5. Moreover, the MNP entrapped in protein can be reused, thereby saving in capital cost and investment of the process and thus supporting the concept of green chemistry.

## Claims

1. A kit comprising uncoated magnetic nanoparticle (MNP) entrapped in a matrix of protein cross-linked with epichlorohydrin, wherein the MNP is entrapped by a method comprising incubating the protein with magnetic nanoparticles in a binding buffer having pH ranging from 6-9 along with epichlorohydrin at a temperature of about 4°C to 30°C, with the optional supplementation of salts, and wherein the MNP is entrapped in a ratio ranging from about 1:5 to 1:0.25 and has a particle size in the range of 1 to 100nm,
wherein:
(A)the kit is a diagnostic kit for immobilization, purification and identification of IgG, Fab fragments or single chain variants, functional proteins from biological samples comprising:
(i) 25µgm to 10mg magnetic nanoparticle entrapped in a matrix of cross-linked protein having affinity to IgG/Fab fragments in 5mM Sodium phosphate buffer, pH 8.0;
(ii) 25ml washing buffer, i.e. IX phosphate buffer pH 8;
(iii) 50ml IgG/Fab fragment elution buffer, i.e. an acidic buffer comprising phosphate buffer pH 2.8; or glycine, pH 2; or L-Arginine, pH 3;
(iv) 2ml neutralizing buffer, i.e. 1M Tris, pH 9.0;
(v) a catalogue comprising instructions and parameters relating to use of the kit; or
(B) the kit is an immunoprecipitation kit for the detection of an immunogen or antigenic proteins comprising
(i) 25µg to 10 mg magnetic nanoparticle entrapped in a matrix of cross-linked protein having affinity to IgG/Fab fragments in 5mM Sodium phosphate buffer, pH 8.0;
(ii) a primary antibody specific to antigen to be detected;
(iii) 1ml of IX Sodium Dodecyl Sulfate (SDS) buffer;
(iv) micro-centrifuge tubes;
(v) a catalogue comprising instructions and parameters relating to use of the kit; and
wherein
(vi) the proteins are selected from the group consisting of ProteinA-MNP, ProteinG-MNP, Protein L-MNP, peroxidase-MNP, glutathione-MNP, Bovine serum albumin (BSA)-MNP, ovalbumin-MNP, amylase-MNP, hemoglobin-MNP, lipase-MNP, Fab-MNP, ScFv-MNP, IgG-MNP, lectin-MNP, calmodulin-MNP, streptavidin-MNP, Albumin-MNP, gelatin-MNP, histone-MNP; and
(vii) the biological samples are selected from the group consisting of biological fluids selected from whole blood, serum, plasma and ascites; cell culture and bacterial cell lysate.

2. The kit according to claim 1, wherein incubation is carried for 1 to 72 hours.

3. The kit according to claim 1, wherein the magnetic nanoparticles are selected from the group consisting of magnetite, ulvospinel, hematite, ilmenite, maghemite, jacobsite, trevorite, magnesioferrite, pyrrhotite, greigite, troilite, goethite, lepidocrocite, feroxyhyte, iron, nickel, cobalt, awaruite, wairauite, or any combination thereof.

4. The kit according to claim 1, wherein the binding buffer is selected from a Phosphate, Carbonate, Borate and combinations thereof, with molar concentrations ranging from 5 mM to 200 mM.

5. The kit according to claim 1, wherein the cross-linking agent is epichlorohydrin at a concentration of from 0.1M to 2M.

6. The kit according to claim 1, wherein the salts are selected from the group consisting of sodium chloride, potassium chloride, calcium chloride, magnesium chloride and any combination thereof.

7. Use of the diagnostic kit of any one of claims 1-6 in a method for identification and purification of IgG, Fab fragments or single chain variants the said method comprising;
(i) diluting the biological sample with phosphate buffer and treating it with cross-linked Protein A-MNPs synthesized and subsequently subjecting the sample to rotation for about 2hrs at room temperature;
(ii) washing the nanoparticles after rotation in step (i) at least twice with phosphate buffer and eluting the antibody bound to the Protein A-magnetic nanoparticles with an acidic buffer; and
(iii) neutralizing the antibody with 1M Tris buffer, pH9 and characterizing the IgG by gel electrophoresis technique followed by Western blotting.

8. Use of the immunoprecipitation kit of any one of claims 1-6 in a method for the detection of an immunogen or antigenic proteins, the said method comprising:
(i) treating a cell lysate with primary antibody specific to the protein depending on the requirements of the experiment to be detected followed by addition of magnetic nanoparticle entrapped in protein to form aAg-primary antibody-protein-MNP complex; and
(ii) heating the Ag-primary antibody-protein-MNP complex of step (i) up to 100°C, followed by running the mixture on SDS PAGE under reducing conditions to identify the Ag.

## Patentansprüche

1. Kit, der unbeschichtetes magnetisches Nanopartikel (MNP) umfasst, das in einer Matrix von Protein, das mit Epichlorhydrin vernetzt ist, eingeschlossen ist, wobei das MNP durch ein Verfahren eingeschlossen wird, das das Inkubieren des Proteins mit magnetischen Nanopartikeln in einem Bindungspuffer, der einen pH im Bereich von 6-9 aufweist, zusammen mit Epichlorhydrin bei einer Temperatur von etwa 4 °C bis 30 °C, mit der optionalen Ergänzung von Salzen, umfasst und wobei das MNP in einem Verhältnis im Bereich von etwa 1 : 5 bis 1 : 0,25 eingeschlossen wird und eine Partikelgröße im Bereich von 1 bis 100 nm aufweist,
wobei:
(A) der Kit ein diagnostischer Kit für die Immobilisierung, Aufreinigung und Identifizierung von IgG, Fab-Fragmenten oder Einzelkettenvarianten, funktionellen Proteinen aus biologischen Proben ist, der Folgendes umfasst:
(i) 25 µg bis 10 mg magnetische Nanopartikel, die in einer Matrix von vernetztem Protein eingeschlossen sind, das eine Affinität zu IgG/Fab-Fragmenten aufweist, in 5 mM Natriumphosphatpuffer, pH 8,0;
(ii) 25 ml Waschpuffer, d. h. 1 x Phosphatpuffer pH 8;
(iii) 50 ml IgG/Fab-Fragment-Elutionspuffer, d. h. einen sauren Puffer, der Phosphatpuffer, pH 2,8, oder Glycin, pH 2, oder L-Arginin, pH 3, umfasst;
(iv) 2 ml Neutralisationspuffer, d. h. 1 M Tris, pH 9,0;
(v) ein Verzeichnis, das Anweisungen und Parameter in Bezug auf die Verwendung des Kits umfasst;
oder
(B) der Kit ein Immunpräzipitations-Kit für den Nachweis eines Immunogens oder von antigenen Proteinen ist, der Folgendes umfasst:
(i) 25 µg bis 10 mg magnetische Nanopartikel, die in einer Matrix von vernetztem Protein eingeschlossen sind, das eine Affinität zu IgG/Fab-Fragmenten aufweist, in 5 mM Natriumphosphatpuffer, pH 8,0;
(ii) einen primären Antikörper, der für das nachzuweisende Antigen spezifisch ist;
(iii) 1 ml von 1 x Natriumdodecylsulfat (SDS)-Puffer;
(iv) Mikrozentrifugenröhrchen;
(v) ein Verzeichnis, das Anweisungen und Parameter in Bezug auf die Verwendung des Kits umfasst;
und
wobei
(vi) die Proteine aus der Gruppe ausgewählt sind, die aus Folgenden besteht: Protein-A-MNP, Protein-G-MNP, Protein-L-MNP, Peroxidase-MNP, Glutathion-MNP, Rinderserumalbumin (BSA)-MNP, Ovalbumin-MNP, Amylase-MNP, Hämoglobin-MNP, Lipase-MNP, Fab-MNP, ScFv-MNP, IgG-MNP, Lektin-MNP, Calmodulin-MNP, Streptavidin-MNP, Albumin-MNP, Gelatine-MNP, Histon-MNP; und
(vii) die biologischen Proben aus der Gruppe ausgewählt sind, die aus biologischen Flüssigkeiten, die aus Vollblut, Serum, Plasma und Aszites ausgewählt sind; Zellkultur und Bakterienzelllysat besteht.

2. Kit nach Anspruch 1, wobei die Inkubation für 1 bis 72 Stunden durchgeführt wird.

3. Kit nach Anspruch 1, wobei die magnetischen Nanopartikel aus der Gruppe ausgewählt sind, die aus Folgenden besteht: Magnetit, Ulvöspinell, Hämatit, Ilmenit, Maghemit, Jacobsit, Trevorit, Magnesioferrit, Pyrrhotit, Greigit, Troilit, Goethit, Lepidokrokit, Feroxyhyt, Eisen, Nickel, Cobalt, Awaruit, Wairauit oder jedweder Kombination davon.

4. Kit nach Anspruch 1, wobei der Bindungspuffer aus einem Phosphat, Carbonat, Borat und Kombinationen davon, mit molaren Konzentrationen im Bereich von 5 mM bis 200 mM, ausgewählt ist.

5. Kit nach Anspruch 1, wobei das Vernetzungsmittel Epichlorhydrin bei einer Konzentration von 0,1 M bis 2 M ist.

6. Kit nach Anspruch 1, wobei die Salze aus der Gruppe ausgewählt sind, die aus Natriumchlorid, Kaliumchlorid, Calciumchlorid, Magnesiumchlorid und jedweder Kombination davon besteht.

7. Verwendung des diagnostischen Kits nach einem der Ansprüche 1-6 in einem Verfahren für die Identifizierung und Aufreinigung von IgG, Fab-Fragmenten oder Einzelkettenvarianten, wobei das Verfahren Folgendes umfasst:
(i) Verdünnen der biologischen Probe mit Phosphatpuffer und Behandeln dieser mit synthetisierten vernetztes-Protein-A-MNPs und anschließendes Unterziehen der Probe der Rotation für etwa 2 Std. bei Raumtemperatur;
(ii) Waschen der Nanopartikel nach der Rotation im Schritt (i) mindestens zweimal mit Phosphatpuffer und Eluieren des Antikörpers, der zu den Protein-A-magnetischen-Nanopartikeln gebunden ist, mit einem sauren Puffer; und
(iii) Neutralisieren des Antikörpers mit 1 M Tris-Puffer, pH 9, und Charakterisieren des IgG durch Gelelektrophorese-Technik, gefolgt von Western-Blotting.

8. Verwendung des Immunpräzipitations-Kits nach einem der Ansprüche 1-6 in einem Verfahren für den Nachweis eines Immunogens oder von antigenen Proteinen, wobei das Verfahren Folgendes umfasst:
(i) Behandeln eines Zelllysats mit primärem Antikörper, der für das nachzuweisende Protein, in Abhängigkeit von den Anforderungen des Experiments, spezifisch ist, gefolgt von der Zugabe von magnetischen Nanopartikeln, die in Protein eingeschlossen sind, um einen Ag-primärer-Antikörper-Protein-MNP-Komplex zu bilden; und
(ii) Erhitzen des Ag-primärer-Antikörper-Protein-MNP-Komplexes von Schritt (i) auf bis zu 100 °C, gefolgt vom Laufen der Mischung auf SDS-PAGE unter reduzierenden Bedingungen, um das Ag zu identifizieren.

## Revendications

1. Kit comprenant des nanoparticules magnétiques (MNP) non revêtues piégées dans une matrice de protéine réticulée par de l'épichlorhydrine, dans lequel les MNP sont piégées par une méthode comprenant l'incubation de la protéine avec des nanoparticules magnétiques dans un tampon de fixation ayant un pH dans la plage de 6-9 conjointement avec de l'épichlorhydrine, à une température allant d'environ 4°C à 30°C, avec une complémentation facultative en sels, et dans lequel les MNP sont piégées selon un rapport allant d'environ 1:5 à 1:0,25 et présentent une taille de particules dans la plage allant de 1 à 100 nm,
où :
(A) le kit est un kit de diagnostic destiné à l'immobilisation, la purification et l'identification d'IgG, de fragments Fab ou de variants à chaîne unique, de protéines fonctionnelles issus d'échantillons biologiques comprenant
(i) de 25 µg à 10 mg de nanoparticules magnétiques piégées dans une matrice de protéine réticulée ayant une affinité vis-à-vis d'IgG/fragments Fab dans un tampon de phosphate de sodium à 5 mM, pH 8,0 ;
(ii) 25 ml de tampon de lavage, c'est-à-dire du tampon phosphate 1X, pH 8 ;
(iii) 50 ml de tampon d'élution d'IgG/fragment Fab, c'est-à-dire un tampon acide comprenant du tampon phosphate, pH 2,8 ; ou glycine, pH 2 ; ou L-arginine, pH 3 ;
(iv) 2 ml de tampon de neutralisation, c'est-à-dire Tris 1M, pH 9,0 ;
(v) un catalogue comprenant des instructions et des paramètres liés à l'utilisation du kit ;
ou
(B) le kit est un kit d'immunoprécipitation destiné à la détection d'un immunogène ou de protéines antigéniques comprenant
(i) de 25 µg à 10 mg de nanoparticules magnétiques piégées dans une matrice de protéine réticulée ayant une affinité vis-à-vis d'IgG/fragments Fab dans un tampon de phosphate de sodium à 5 mM, pH 8,0 ;
(ii) un anticorps primaire spécifique de l'antigène à détecter ;
(iii) 1 ml de tampon de dodécylsulfate de sodium (SDS) 1X ;
(iv) des tubes de micro-centrifugation ;
(v) un catalogue comprenant des instructions et des paramètres liés à l'utilisation du kit ; et
où
(vi) les protéines sont choisies dans le groupe constitué par la Protéine A-MNP, la Protéine G-MNP, la Protéine L-MNP, la peroxydase-MNP, le glutathion-MNP, l'albumine de sérum bovin (BSA)-MNP, l'ovalbumine-MNP, l'amylase-MNP, l'hémoglobine-MNP, la lipase-MNP, un Fab-MNP, un ScFv-MNP, l'IgG-MNP, la lectine-MNP, la calmoduline-MNP, la streptavidine-MNP, l'albumine-MNP, la gélatine-MNP, l'histone-MNP, et
(vii) les échantillons biologiques sont choisis dans le groupe constitué par les fluides biologiques choisis parmi le sang entier, le sérum, le plasma et l'ascite ; une culture cellulaire et un lysat de cellules bactériennes.

2. Kit selon la revendication 1, dans lequel l'incubation est effectuée pendant de 1 à 72 heures.

3. Kit selon la revendication 1, dans lequel les nanoparticules magnétiques sont choisies dans le groupe constitué par la magnétite, l'ulvospinelle, l'hématite, l'ilménite, la maghémite, la jacobsite, la trévorite, la magnésioferrite, la pyrrhotite, la greigite, la troïlite, la goethite, la lépidocrocite, le feroxyhyte, le fer, le nickel, le cobalt, l'awaruite, la wairauite, ou une combinaison quelconque de ceux-ci.

4. Kit selon la revendication 1, dans lequel le tampon de fixation est choisi parmi un phosphate, un carbonate, un borate et des combinaisons de ceux-ci, ayant des concentrations molaires allant de 5 mM à 200 mM.

5. Kit selon la revendication 1, dans lequel l'agent de réticulation est l'épichlorhydrine selon une concentration allant de 0,1 M à 2 M.

6. Kit selon la revendication 1, dans lequel les sels sont choisis dans le groupe constitué par le chlorure de sodium, le chlorure de potassium, le chlorure de calcium, le chlorure de magnésium et une combinaison quelconque de ceux-ci.

7. Utilisation du kit de diagnostic selon l'une quelconque des revendications 1-6 dans une méthode d'identification et de purification d'IgG, de fragments Fab ou de variants à chaîne unique, ladite méthode comprenant :
(i) la dilution de l'échantillon biologique par du tampon phosphate et son traitement par des MNP-protéine A réticulée synthétisées, puis la soumission de l'échantillon à une rotation pendant environ 2 h à température ambiante ;
(ii) le lavage des nanoparticules après la rotation dans l'étape (i) au moins deux fois par du tampon phosphate et l'élution de l'anticorps lié aux nanoparticules magnétiques-protéine A par un tampon acide ; et
(iii) la neutralisation de l'anticorps par du tampon Tris 1M, pH 9, et la caractérisation de l'IgG par une technique d'électrophorèse sur gel, suivie d'un buvardage de Western.

8. Utilisation du kit d'immunoprécipitation selon l'une quelconque des revendications 1-6 dans une méthode de détection d'un immunogène ou de protéines antigéniques, ladite méthode comprenant :
(i) le traitement d'un lysat cellulaire par un anticorps primaire spécifique de la protéine, en fonction des exigences de l'expérience, à détecter suivi de l'addition de nanoparticules magnétiques piégées dans une protéine, afin de former un complexe Ag-anticorps primaire-protéine-MNP ; et
(ii) le chauffage du complexe Ag-anticorps primaire-protéine-MNP de l'étape (i) jusqu'à 100°C, suivi de l'analyse du mélange par SDS-PAGE en conditions réductrices, afin d'identifier l'Ag.
